(19) Europäisches Patentamt

European Patent Office

Office européen des brevets

(11) EP 0 638 134 B1

## (12) EUROPEAN PATENT SPECIFICATION

(45) Date of publication and mention
of the grant of the patent:
**11.12.1996  Bulletin 1996/50**

(21) Application number: **93911892.3**

(22) Date of filing: **29.04.1993**

(51) Int Cl.6: **D04H 1/54**, D04H 1/42,
D04H 1/48

(86) International application number:
**PCT/GB93/00897**

(87) International publication number:
**WO 93/22486 (11.11.1993 Gazette 1993/27)**

(54) **ABSORBENT MATERIAL AND A METHOD OF MAKING SAME**

ABSORBIERENDES MATERIAL UND VERFAHREN ZU DESSEN HERSTELLUNG

MATERIAU ABSORBANT ET PROCEDE DE FABRICATION

(84) Designated Contracting States:
**BE CH DE ES FR GB IT LI NL PT SE**

(30) Priority: **01.05.1992  GB 9209463**

(43) Date of publication of application:
**15.02.1995  Bulletin 1995/07**

(73) Proprietors:
- **USM ESPANA, S.L.**
  **E-08027 Barcelona (ES)**
  Designated Contracting States:
  **ES**
- **BRITISH UNITED SHOE MACHINERY LIMITED**
  **Belgrave Leicester LE4 5BX (GB)**
  Designated Contracting States:
  **BE CH DE FR GB IT LI NL PT SE**

(72) Inventors:
- **BRASSINGTON, Nigel, John Greenacre**
  **East Parade**
  **Saltburn-by-the-Sea Cleveland TS12 2BJ (GB)**
- **WELBURN, Jean 47 Marway Road**
  **Saltburn-by-the-Sea Cleveland TS12 2RH (GB)**

(74) Representative: **Atkinson, Eric et al**
**c/o USM TEXON LTD**
**Patent & Legal Department**
**P.O. Box 88**
**Ross Walk**
**Belgrave**
**Leicester LE4 5BX (GB)**

(56) References cited:
**EP-A- 0 388 062**       **EP-A- 0 388 072**
**EP-A- 0 396 296**       **US-A- 5 104 703**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Technical Field

This invention is concerned with an absorbent material and method for making same and is especially concerned with absorbent materials suitable for use in medical or hygienic absorbence for the absorption of body fluids, for example in surgical dressings, sanitary products and incontinence products.

### Background of the Invention

There are described in EP-A-0 388 062 various absorbent materials of the type comprising a non-woven fibre sheet having a dense surface layer of fibres at each face of the sheet and, between the surface layers, a relatively low density region which, apart from an initial "tacking" operation, has not been subjected to any substantial needling. More particularly the preferred materials there described each comprise at least a major proportion of hydrophilic fibres, although there is also a passing reference therein to the possibility of producing absorbent material entirely of hydrophobic fibres. The preferred materials described in said specification utilised in particular a mixture of hydrophilic and hydrophobic fibres, the hydrophilic fibres serving to absorb fluids while the hydrophobic fibres provided a "scaffolding" for retaining the structure of the low density region, even under compression. In the case of the wholly hydrophobic fibre material, the fluid is believed to have been effectively "stored" within the structure rather than being absorbed into the fibres themselves.

Although the absorbent materials described in detail in said specification have in practice proved generally satisfactory, nevertheless when fluid has been absorbed by the hydrophilic fibres in the web there arises a tendency in these hydrophilic fibres to collapse, especially under compression, so that although the use of hydrophilic fibres is believed to enhance absorbency, absorbent webs including hydrophilic fibres may tend to collapse and thus have a less than optimum absorbency and may even "wet back" when a load is applied, that is the absorbed fluid may be forced out of the material by the pressure.

### Objects of the Invention

It is one of the various objects of the present invention to provide an improved absorbent material in which the disadvantages of currently available materials are mitigated.

It is a further one of the various objects to provide an improved method of producing such materials.

### Summary of the Invention

The invention thus provides, in one of its several aspects, an absorbent material comprising a non-woven fibre sheet having a dense surface layer of fibres at each face of the sheet and, between the surface layers, a relatively lower density region in which a substantial majority of the fibres extend generally in the plane of the sheet, the fibres being bonded together, at least to some extent, by a heat-activated bonding agent in fibrous form which remains in fibrous form in the sheet.

The invention further provides, in another of its several aspects, a method of making an absorbent material comprising forming a non-woven fibre web comprising a blend of fibres including a minor weight of heat-activated bonding agent in fibrous form, subjecting the web to needling at a low punch density with the needles penetrating completely through the web, then subjecting surface regions of the web to needling at a much higher punch density whereby to form a dense surface layer of fibres at each face of the web, and thereafter subjecting the web to heat in such a manner as to activate the bonding agent and cause it to bond to adjacent fibres but without destroying its fibrous structure and without substantially affecting the other fibres of the web and without causing significant shrinkage.

In the material and the method in accordance with the invention set out respectively in the last two preceding paragraphs the bonding agent preferably comprises a hot melt bonding agent. In a preferred embodiment, moreover, the bonding agent is originally (i.e. prior to melting) constituted by one component of a bi-component fibre, preferably of the core/sheath type, in which said one (the sheath) component has a lower softening point than the other (core) component, the latter remaining in fibrous form after heat activation of the one component. However, other suitable bonding agents may be used provided that their fibrous form is retained after bonding; for example a fibre component having a fairly low melting point relative to the other components, e.g. polyethylene or polypropylene single component fibres, may be used but will require careful control of bonding conditions during manufacture to ensure sufficient heating to effect bonding whilst still not destroying their fibrous nature.

Preferably, in carrying out a method in accordance with the invention to produce absorbent materials in accordance with the invention, a light non-woven fibre web is produced continuously by known methods, for example using carding techniques, and the light web so produced is cross-lapped to provide a web with a plurality of layers; preferably the web has between 4 and 40 layers, more preferably between 6 and 20 layers, and most preferably between 8 and 14 layers. The web is then tacked completely through by subjecting the web to a needling operation at a low punch density suitably between $\frac{1}{2}$ and 20 per cm$^2$, preferably between 1 and 8 per cm$^2$ and most preferably at a punch density of between 2 and 6 per cm$^2$. Surface regions of the web are thereafter subjected to needling at a much higher punch density, suitably between 100 and 1,000 per cm$^2$, preferably between 200 and 600 per cm$^2$ and more preferably between 300 and 500 per cm$^2$, to form a dense surface layer of fibres at each face of the web. The high density needling may conveniently be arranged to penetrate each surface of the web to no more than 15% of the thickness of the web prior to that needling, for example to a depth of not more than 0.5mm.

In carrying out a preferred method in accordance with the invention, the fabric is subjected to heat suitably by blowing hot air through the web in sufficient volume and for a sufficient period of time to soften the bonding agent, e. g. a sheath component where a bi-component core/sheath fibre is used, without having any substantial effect on the remainder of the fibres in the web and without causing any significant shrinkage of the web. It is important that the air flow does not collapse the web or reduce its thickness to any significant degree. In a preferred embodiment, using a bi-component fibre of the core/sheath type, where the core component has a softening point of about 220°C and the sheath a softening point of about 100°C, it has been found sufficient to subject the web to a flow of air at about 175°C for a period of about 1 minute. If desired, after the hot air treatment the web may be subjected to surface rolling suitably carried out at an elevated temperature similar to that of the hot air, conveniently by passing the web between rolls set at a fixed gap; such gap would preferably be fixed to be not less than 70% of one thickness of the web prior to passing through the gap. Such hot rolling will tend to provide an absorbent material with a relatively smooth and lint-free surface which is important in some applications. Cold rolling of the web, using a fixed gap may, however, be useful in some circumstances as this tends to lead to more rapid setting of a hot-melt component which may lead to a somewhat stronger but less absorbent product.

In the manufacture of material in accordance with the invention, preferably the web is formed from a blend of coarse and fine deciTex fibres, the coarse deciTex fibres suitably having a deciTex between 5 and 15, preferably between 5 and 7 deciTex, and the fine deciTex fibre suitably being less than 3 deciTex. The fibres of the bonding agent are preferably fine fibres. The coarse fibres suitably comprise 5 to 40% of the blend, preferably 10 to 30% of the blend and more preferably 15 to 25% of the blend by weight, the balance being fine fibres. In one preferred material the fibre blend suitably includes 2 to 50% of fine bi-component fibre of the core/sheath type in which the sheath has a lower softening point than the core, preferably 3 to 20% and more preferably 5 to 10% by weight. In such blend, furthermore, three fibres are used, preferably all being polyester fibres, namely a major proportion of a fine single component polyester fibre providing a basic structure of the fabric, a major proportion of the remainder of the fibre being a coarse single component polyester fibre and the balance of the fibre being a fine bi-component polyester fibre of the type referred to.

Appropriate fibre lengths must also be selected and these will depend to some extent on the processing techniques to be utilised. Fibre lengths between 20mm and 120mm may be suitable, preferably between 25mm and 90mm and more preferably between 30mm and 70mm.

In a preferred method in accordance with the invention the web weight before subjecting the web to heat is between 70 and 1,000 grammes per square metre, preferably between 80 and 600 grammes per square metre and more preferably between 100 and 400 grammes per square metre. When subjecting the web to heat treatment the change in area of the web is suitably no more than plus or minus 10%, preferably no more than plus or minus 5% and most preferably 0%. The reduction in thickness of the web after heat treatment (and rolling where used) is preferably between 0 and 40% and more preferably between 5 and 20%.

Although materials containing hydrophilic fibres exhibit improved absorbency and strength when produced by a method in accordance with the present invention, it is preferable that the materials in accordance with the invention are constituted wholly by hydrophobic fibres, more particularly fibres which are inherently hydrophobic throughout. Fibres which have been rendered hydrophobic by applying a surface coating are generally less suitable, especially where the coating tends to interfere with bonding.

In another aspect the invention may be considered to provide an absorbent material comprising a non-woven fibre sheet substantially of hydrophobic fibres, the sheet having a dense surface layer of fibres at each face of the sheet and, between the surface layers, a relatively lower density region in which a substantial majority of the fibres extend generally in the plane of the sheet and a small number of fibres extend in a direction generally transverse to the plane of the sheet, the transversely extending fibres having been substantially produced by needle punching at a low punch density and the surface layers having been formed by needle punching at a high punch density.

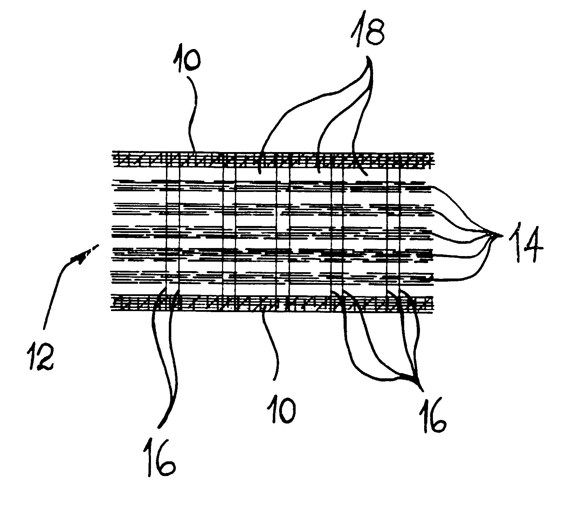

There now follows a detailed description to be read with reference to the accompanying drawing of an absorbent sheet material embodying the invention and a method of making same, itself embodying the invention in its method aspects. It will be realised that this material and method have been selected for description to illustrate the invention by way of example.

Brief Description of the Drawing

The accompanying drawing is a diagrammatic view in section of a portion of an absorbent material embodying the invention.

Description of the Preferred Embodiment

The illustrative material comprises a non-woven sheet having a dense surface layer (10) of fibres at each surface and, between the surface layers (10), a relatively lower density region in which a substantial majority of the fibres extend generally in the plane of the sheet. This lower density region (12) comprises a plurality of layers (14) formed by cross-lapping as hereinafter described. A small number of fibres (16) extend in a direction generally transverse to the plane of the sheet, the transversely extending fibres (16) having been produced, in the manufacture of the illustrative absorbent material, by needle punching at a relatively low punch density and the relatively dense surface layers (10) having been formed by needle punching at a higher punch density. The fibres of the absorbent material are bonded together to some extent by a hot melt bonding material originating as a sheath component of a core/sheath type bi-component fibre, the core component remaining in fibrous form in the finished sheet. Methods of manufacturing the illustrative absorbent material referred to above are set out in the following examples.

EXAMPLE I

The following blend of three fibres was used:

| | |
|---|---|
| 76% by weight | 1.7 dTex hydrophobic polyester. This fine dTex fibre provides the basic structure of the fabric, giving rise to a large number of interstices. It also provides loft and softness to handle. The particular fibre used was Trevira type 290, which has a melt temperature of 230°C. |
| 19% by weight | 5.0 dTex hydrophobic polyester. This coarse dTex fibre is believed to provide scaffolding, i.e. to act as a structural element which will tend to oppose any mechanical forces acting on the fabric. The particular fibre used was also Trevira type 290. |
| 5% by weight | Fine dTex (about 2.2 dTex) bi-component core/sheath polyester, the melt temperature of the core being about 220°C and of the sheath about 120°C. The particular fibre used was Trevira type 252. |

These fibres were blended in the stated proportions prior to being processed through high-efficiency cards to produce an open web of about 12 grammes per square metre. This resultant web was then cross-lapped to give a web with 11 laps (layers).

The web formed by cross-lapping these layers was then tacked completely through using a needle punch density of approximately $4/cm^2$. High efficiency 40 gauge tacking needles were used. This reduced the loft of the web considerably and provided fibres in the direction generally transverse to the plane of the web which held the layers together.

The resultant tacked web was then needled through two looms, in such a way that the needle barbs had a significant densifying effect only at the surfaces of the webs, i.e. low needle penetrations were used. High efficiency 40 gauge needles were used throughout. Loom 1 provided a needle punch density, on the top surface only, of $120/cm^2$. Loom 2 provided a needle punch density, on both top and bottom surfaces, of $180/cm^2$. The penetration for the lower needles used on loom 2 was slightly greater than the top needle penetration, to compensate for the top surface needling on loom 1. The intention was to provide a web with top and bottom surfaces substantially equally densified. The web at this stage had a weight of about 170 grammes per square metre and a loft of around 4mm.

The web was then through-air bonded before being very lightly surface-rolled. The through-air bonding was carried out at 175°C with an air flow of 132 m/min and a dwell time of 1 min. This air flow was such that the bi-component fibre rapidly reached the melt temperature of its sheath component without causing significant shrinkage and without collapsing the web or reducing its thickness significantly. The area change through the heat treatment process was about +4%. The subsequent surface rolling was carried out at 175°C using heated rolls set 3mm apart. After rolling the fabric was rapidly cooled by drawing cold air through the web. The fabric at this stage had a weight of 165 grammes per square metre and an unconstrained thickness of around 3.5mm.

The finished fabric had the following apparent properties (initial measurements):

```
Typical free absorbency (no load)          2050%    20.5 g/g
Absorbency under load (135g/100cm2)        1600%    16 g/g
```

```
Absorbency under load (285g/100cm²)        1500%    15 g/g

Weight 165 gsm                             Thickness 3.5mm
```

The illustrative absorbent material of a thickness as set out in this Example I has good drape and handle.

It is thought that between the layers (14) air spaces (18) may exist, contributing to the absorbency volume: it is important in processing to ensure that the sheet material is not caused to collapse.

EXAMPLE II

Example I was repeated, as closely as possible, except that a different 1.7 dTex polyester fibre was used. This fibre was sourced from Tuntex (Thailand). This constitutes 76% of the fibre blend. It was found that the resultant fabric and properties were much the same as Example I above.

EXAMPLE III

This Example illustrates the use of a melt fibre that is not bi-component in structure. In this case an amorphous thermoformable fibre, viz. polypropylene, was used. Similar results could be obtained using other melt fibres, e.g. polyethylene and low melting point polyester.

The following blend of fibres was used:

60% by weight   1.7 dTex polyester of the Trevira type T290
20% by weight   5.0 dTex polyester, also of the Trevira T290 type
20% by weight   2.8 dTex polypropylene. This fibre constitutes the fusible fibre of the structure and has a melt temperature in the order of 160°C. The particular fibre used was Moplefan type CS2.

As with Examples I and II, these fibres were blended in the stated proportions prior to being processed through high-efficiency cards and the resultant web was cross-lapped. Tacking then took place as previously described using 40 gauge tacking needles. Thereafter the tacked web was needled in a first loom to a penetration, from top and bottom, of 8mm and with a punch density of $100/cm^2$ to give structure and strength to the material, and thereafter in Loom 2 needling took place to a penetration of 4.8mm (top) and 6.2mm (bottom) with a punch density of $300/cm^2$ thus to provide the denser surface layers. At this stage the web had a weight of about 170 gsm and a loft of 2.4mm.

As in Examples I and II, the web was then through-air bonded, at a temperature of 180°C and a dwell time of 1 minute; in this case, however, no surface-rolling took place. The throughput of air was such that the polypropylene fibres melted at least superficially to enable them to flow so that they bonded with adjacent fibres and, upon cooling, effectively locked them in position, but without collapsing the web during such heating or reducing substantially its thickness by shrinkage. After heating, the web was shock-cooled by drawing cold air therethrough. The finished product had a weight of 180 gsm and an unconstrained thickness (loft) of about 2.2mm, with good tensile strength and a free absorbency (no load) in the order of 1200% (12g/g).

COMPARATIVE EXAMPLE

Example I was repeated, but omitting the bi-component polyester from the blend. The blend used 80% 1.7 dTex polyester and 20% 5.0 dTex polyester. The resultant fabric, although still absorbent, showed a much reduced absorbency and physical strength.

In the case of the materials in accordance with the invention made as described in Examples I, II and III above, it has been found that despite the weakly bound nature of the material as a whole, the bonding of the fibres resists the tendency of the structure to collapse when wetted and thus reduces any tendency to "wet back". In the particular Examples, moreover, despite the hydrophobic nature of the fibres used, the absorbing properties are surprisingly good. Absorbency and retentivity are of a level normally associated with hydrophilic fabrics, e.g. those made with significant quantities of rayon or cotton which, however, as mentioned above, can be subject to wet collapse. As can be seen from the test results above, the illustrative absorbent material performs very satisfactorily when the wet material is subjected to loads.

Where the absorbent material is made entirely of hydrophobic fibres, furthermore, the surfaces thereof feel surprisingly dry even when a considerable amount of fluid is retained within the material: it is believed that this arises from the increased surface density of the fabric and from the tendency to preferentially wick fluid into the low density core region away from the surfaces. It is common practice in other absorbent materials to apply a separate non-woven fabric to provide such a "stay-dry" effect: this separate material may be eliminated in the case of the illustrative absorbent materials.

The surfaces of the surface layers (10) of the illustrative absorbent materials are relatively smooth and lint-free which are distinct advantages for fabrics for use in surgical and catamenial applications. Furthermore the illustrative materials provide good mechanical cushioning and resilience which may improve comfort in some products. It is believed that the bonding provided contributes to the increase in strength and resiliency as well as the relatively smooth surface.

An additional advantage of the illustrative absorbent materials where made entirely of hydrophobic fibres, arises in that the cost of such fibres tends to be noticeably cheaper than corresponding hydrophilic fibres, e.g. cotton and rayon, and such fibres are often easier to process.

Furthermore, absorption of fluids as occurs into hydrophilic fibres may be disadvantageous for some products, e.g. surgical, catamenial or incontinence products intended to be reused. Where materials including hydrophilic fibres are included in re-usable absorbent products which are intended to be washed before re-use, the fibres themselves absorb a certain amount of material and this material is not entirely removed from the hydrophilic fibres by washing. This can lead to residual odour and the possibility of cross-infection. Hydrophobic fibres cannot absorb aqueous, e.g. body, fluids and so washing the absorbent material is able to remove all or substantially all of the absorbed materials because the absorbed materials are absorbed purely interstitially.

## Claims

1. An absorbent material comprising a non-woven fibre sheet having a dense surface layer of fibres at each face of the sheet and, between the surface layers, a relatively lower density region in which a substantial majority of the fibres extend generally in plane of the sheet, the fibres being bonded together, at least to some extent, by a heat-activated bonding agent in fibrous form which remains in fibrous form in the sheet.

2. An absorbent material according to Claim 1 wherein the bonding agent comprises a hot melt bonding material originating as one component of a bi-component fibre of the type referred to, the other component remaining in fibrous form in the sheet.

3. A material according to Claim 1 wherein the bonding agent is constituted by polypropylene fibres.

4. A material according to Claim 1 comprising a blend of fibres including 5 to 40% by weight coarse deciTex fibres, i.e. of at least 5 deciTex, the balance being fine deciTex fibres, i.e. of less than 3 deciTex.

5. A material according to any one of the preceding Claims where the fibre layers are constituted substantially wholly by hydrophobic fibres.

6. A material according to Claim 1 wherein the surface layers are less than 0.5mm in thickness.

7. A material according to Claim 1 wherein the low density region provides at least 70% of the thickness of the sheet material.

8. A method of making an absorbent material comprising forming a non-woven fibre web comprising a blend of fibres including a minor weight of a heat-activated bonding agent in fibrous form, subjecting the web to needling at a low punch density with the needles penetrating through the web, then subjecting surface regions only of the web to needling at a much higher punch density whereby to form a dense surface layers of fibres at each face of the web, and thereafter subjecting the web to heat in such a manner as to activate the bonding agent and cause it to bond to adjacent fibres but without destroying its fibrous structure and without substantially affecting the other fibres of the web and without causing significant shrinkage.

9. A method according to Claim 8 wherein the web is subjected to heat by passing hot air through the web in sufficient volume and for a suitable period of time.

10. A method according to Claim 9 comprising passing the web, after subjecting the web to heat, between rolls set at a fixed gap.

11. A method according to Claim 9 wherein the web is cooled after having been subjected to heat by passing cool air through the web.

12. A method according to Claim 8 wherein the low punch density is from $\frac{1}{2}$ to 20 per cm$^2$ and the high punch density from 100 to 1,000 per cm$^2$.

**Patentansprüche**

1. Ein Absorptionsmaterial, das einen Faservliesstoff mit einer dichten Faser-Oberflächenschicht auf jeder Seite des Stoffes umfaßt und einen Bereich relativ niedriger Dichte zwischen den Oberflächenschichten, in welchem sich eine wesentliche Mehrheit der Fasern allgemein in der Ebene des Stoffes erstreckt. wobei die Fasern zumindest in gewissem Maß durch ein wärmeaktiviertes Haftmittel in Faserform, welches im Stoff in Faserform verbleibt, verbunden werden.

2. Ein Absorptionsmaterial gemäß Anspruch 1,
in dem das Haftmittel ein Heißschmelz-Haftmaterial umfaßt, das als eine Komponente aus einer Zweikomponentenfaser des Typs stammt, auf den Bezug genommen wird, wobei die andere Komponente in Faserform im Stoff verbleibt.

3. Ein Material gemäß Anspruch 1,
in welchem das Haftmittel durch Polypropylenfasern gebildet wird.

4. Ein Material gemäß Anspruch 1,
das eine Mischung aus Fasern mit grobem Dezitex, d. h. mit mindestens 5 Dezitex, für 5 bis 40 % des Gewichts umfaßt, wobei der Rest Fasern mit feinem Dezitex, d. h. mit weniger als 3 Dezitex, sind.

5. Ein Material gemäß einem der vorgenannten Ansprüche,
in dem die Faserschichten im wesentlichen komplett durch hydrophobe Fasern gebildet werden.

6. Ein Material gemäß Anspruch 1,
in welchem die Oberflächenschichten eine Dicke von weniger als 0,5 mm haben.

7. Ein Material gemäß Anspruch 1,
in welchem der Bereich geringer Dichte mindestens 70 % der Dicke des Stoffes ausmacht.

8. Ein Verfahren zur Herstellung eines Absorptionsmaterials,
welches das Formen eines Faservliesgewebes aus einer Fasermischung mit einem geringeren Gewichtsanteil von wärmeaktiviertem Haftmittel in Faserform umfaßt, das Ausüben eines Nadelvorgangs auf das Gewebe bei niedriger Nadeldichte, bei welchem die Nadeln das Gewebe durchdringen, dann das Ausüben eines Nadelvorgangs nur auf die Oberflächenbereiche des Gewebes bei einer viel höheren Nadeldichte, um dadurch eine dichte Faser-Oberflächenschicht auf jeder Seite des Gewebes zu bilden und anschließend das Einwirken von Wärme auf das Gewebe, um das Haftmittel zu aktivieren und zu bewirken, daß es mit den danebenliegenden Fasern verbunden wird, jedoch ohne die Faserstruktur des Haftmittels zu zerstören und ohne die anderen Fasern des Gewebes wesentlich zu beeinträchtigen und ohne wesentliche Schrumpfung zu verursachen.

9. Ein Verfahren gemäß Anspruch 8,
bei welchem das Gewebe der Einwirkung von Wärme ausgesetzt wird indem Warmluft in ausreichender Menge und während eines ausreichenden Zeitraums durch das Gewebe geführt wird.

10. Ein Verfahren gemäß Anspruch 9,
welches, nachdem das Gewebe der Einwirkung von Wärme ausgesetzt wurde, das Durchführen des Gewebes zwischen auf einen festgelegten Spalt eingestellte Walzen umfaßt.

11. Ein Verfahren gemäß Anspruch 9,
bei welchem das Gewebe, nachdem es der Einwirkung von Wärme ausgesetzt wurde, gekühlt wird, indem kühle Luft durch das Gewebe geführt wird.

**12.** Ein Verfahren gemäß Anspruch 8,
bei welchem die niedrige Nadeldichte von 1/2 bis 20 pro cm$^2$ und die hohe Nadeldichte von 100 bis 1000 pro cm$^2$ reicht.

**Revendications**

**1.** Matière absorbante composée d'une feuille en fibres non-tissée ayant une couche superficielle dense de fibres au niveau de chaque face de la feuille et, entre les couches superficielles, une région à densité relativement inférieure dans laquelle une grande majorité des fibres s'étendent généralement dans le plan de la feuille, les fibres étant collées ensemble, au moins dans une certaine mesure, par un agent adhésif activé à la chaleur sous forme fibreuse qui demeure sous forme fibreuse dans la feuille.

**2.** Matière absorbante selon la revendication 1, dans laquelle l'agent adhésif comprend une matière adhésive à fusion se présentant à l'origine sous forme de l'un des constituants d'une fibre à deux consistuants du type auquel on fait référence, l'autre constituant demeurant sous forme fibreuse dans la feuille.

**3.** Matière selon la revendication 1, dans laquelle l'agent adhésif est constitué par des fibres de polypropylène.

**4.** Matière selon la revendication 1, composée d'un mélange de fibres incluant de 5 à 40 % en poids de fibres à décitex gros, c'est-à-dire d'au moins 5 décitex, le reste étant des fibres à décitex fin, c'est-à-dire de moins de 3 décitex.

**5.** Matière selon l'une quelconque des revendications précédentes, dans laquelle les couches de fibres sont constituées sensiblement complètement par des fibres hydrophobes.

**6.** Matière selon la revendication 1, dans laquelle les couches superficielles sont inférieures à 0,5mm d'épaisseur.

**7.** Matière selon la revendication 1, dans laquelle la région à faible densité fournit au moins 70% de l'épaisseur de la matière de la feuille.

**8.** Procédé de production d'une matière absorbante comprenant les étapes de : former une nappe en fibres non-tissée composée d'un mélange de fibres comprenant un poids faible d'un agent adhésif activé à la chaleur sous forme fibreuse, soumettre la nappe à un aiguilletage à faible densité de perçage avec les aiguilles pénétrant à travers la nappe, puis soumettre uniquement les régions superficielles de la nappe à un aiguilletage à une densité de perçage beaucoup plus élevée pour ainsi former une couche superficielle dense de fibres au niveau de chaque face de la nappe, et par la suite soumettre la nappe à de la chaleur de sorte à activer l'agent adhésif et à provoquer son adhésion aux fibres adjacentes mais sans destruction de sa structure fibreuse et sans sensiblement affecter les autres fibres de la nappe et sans provoquer de retrait considérable.

**9.** Procédé selon la revendication 8, dans lequel la nappe est soumise à de la chaleur en faisant passer de l'air chaud à travers la nappe en volume suffisant et pendant un laps de temps approprié.

**10.** Procédé selon la revendication 9, comprenant les étapes de faire passer la nappe, une fois qu'elle a été soumise à de la chaleur, entre des cylindres réglés à un écart fixe.

**11.** Procédé selon la revendication 9, dans lequel la nappe est refroidie, après avoir été soumise à de la chaleur, en faisant passer de l'air frais à travers la nappe.

**12.** Procédé selon la revendication 8, dans lequel la faible densité de perçage est de ½ à 20 par cm$^2$ et la densité de perçage élevée de 100 à 1000 par cm$^2$.